# EUROPEAN PATENT APPLICATION

(11) **EP 2 258 343 A1**
(43) Date of publication of application: **08.12.2010**
(21) Application number: 10174847.3
(22) Date of filing: 22.09.2005
(51) Int. Cl.: A61K 9/12, A61M 15/00, A61K 9/00

(54) **Method and kits for delivering drugs by nebulisation**

(30) Priority: 08.10.2004 GB 0422413
(62) Divisional of application: 05784738.6
(71) Applicant: Breath Limited, Biggin Hill, Kent TN16 3TR (GB)
(72) Inventor: McAffer, Ian G. C., Biggin Hill, Kent TN16 3TR (GB); Tasko, Peter, Hitchin, Hertfordshire SG5 1PA (GB)
(74) Representative: Schlich, George William

(57) **Abstract**

Low volume unit dose vials are provided for administering drugs by nebulisation, together with kits comprising unit dose vials, nebulisers and data carriers. The invention avoids problems associated with prior art unit dose vials, including wastage of drug and inappropriate usage.

## Description

The present invention concerns methods for administering a drug by nebulisation, to kits for carrying out the method, to sets of components of such kits and to unit dose vials, for use in the method of the invention, and for use as components of the kits.

The use of nebulisers to administer drugs to the airways of a subject via inhalation is well known, for example in the treatment of asthma. Generally, a standard volume of drug formulation is poured into the chamber of the nebuliser which, in use, generates an aerosol of droplets which are then inhaled. Drugs for delivery by nebulisation may be prepared as pre-mixed formulations or as concentrates which are diluted to the required concentration with a diluent such as saline in the chamber of the nebuliser immediately prior to administration.

Use of unit dose vials (hereinafter referred to as "UDVs") containing a pre-mixed formulation is known to result in more consistent administration of the desired drug and reduction in errors in formulating the drug or administering the correct dosage. For example, sterile, pre-mixed, pre-measured single unit doses of albuterol and ipratroprium bromide and methods for administering them are described in published patent applications US 2003/0191151 and US 2003/0203930, and in US Patent No 6,632,842.

A disadvantage associated with at least one type of nebuliser is that a significant proportion of the drug formulation may be wasted. Typically, standard UDVs contain an amount of drug determined by the assumption that the chamber of the nebuliser will be filled with from 2 to 3ml of formulation. However, a volume of 0.7-1.0ml of the drug formulation may be required simply to fill the dead space in the nebuliser, before any drug is delivered to the user. That is because as there are no universally recognised standards, the design characteristics of nebulisers vary widely and at least in part, the 2/3ml aliquot provided in current UDVs accomodates these design variations. In addition, as nebulisers have a constant output, a significant proportion of the drug formulation is not inhaled to the target organ and is lost either during the exhalation phase of the breathing cycle to the atmosphere before it can be absorbed, or as a result of droplets containing the drug being swallowed.

A further disadvantage of known methods of nebulisation is the risk that the subject receives an inappropriate dose of the drug to be administered. The risk is more significant in inexperienced or vulnerable subjects, who may for example breath only through their nose rather than, as required, through their mouth. This may, to some extent, be counteracted by the use of a mask in children, but it will not compensate for employing the correct technique. These issues are of particular concern in the case of the steroid drugs often delivered via inhalation, but will also be relevant to other classes of drug.

A further disadvantage of known UDVs is that a user may be tempted to use only a portion of the UDV contents and to retain the residue for use on a future occasion. Such practices are dangerous, as the UDVs are usually sterile and not preserved and the residue can readily become contaminated.

According to a first object of the present invention to provide a kit for administering a drug by nebulisation which reduces waste and ensures delivery of the correct drug dose.

Accordingly, the present invention provides a kit for administering a drug comprising a nebuliser including control means for controlling the nebuliser to initiate drug delivery and one or more UDVs wherein the UDVs contain a pre-mixed drug formulation, said formulation having a volume of less than 1ml, preferably 0.5 to 1.0ml, and wherein data storage means is provided which bears data associated with the one or more UDVs and is readable by the control means, whereby, in use, data is read by the control means and permits actuation of drug delivery. In one embodiment of kit according to the invention, the data storage means is incorporated as part of the nebuliser, i.e. an integrated circuit storage device such as a CMOS RAM or other microprocessor-controlled storage element. In specific examples of such kits, the nebulisers may be high efficiency nebulisers, including high efficiency dosometric nebulisers.

In another embodiment, the data storage means is provided as a separate element of the kit and is adapted to be connected to the nebuliser to permit actuation of drug delivery. In this embodiment the data storage means may be a removable CMOS RAM or a data card or computer disc adapted to be read by a corresponding card reader or disc drive incorporated in the nebuliser. In this embodiment it is envisaged that a consignment of UDVs could be supplied with a data storage means loaded with data associated with that particular consignment.

The kits of the invention allow a smaller volume of drug formulation safely to be used than is standard, due to improved efficiency of drug delivery and less dead space in the nebuliser. This advantageously results in significant reduction in drug waste, reduced nebulising time and reduced cost. It is to be understood that although the volumes of formulation contained in UDVs provided in kits according to the invention are smaller than are customarily used, (1.0ml versus 2-3ml) the concentration of active ingredient will generally remain the same as was used in the larger capacity UDVs.

The nature of the nebulisers used as components of kits according to the invention is not critical.

It is, however, preferable that the nebulisers are designed to operate in an efficient manner in the sense of delivering an accurate and reproducible dose of medicament without undue waste. In this regards, a so-called "High Efficiency Nebuliser" that is adapted to deliver drug at a timing that is synchronised with the breathing cycle is preferably used.

In this regard, a number of types of known designs of nebulisers (including adaptive aerosol delivery nebulisers and dosometric nebulisers) can be incorporated as components of kits according to the invention. One type of high efficiency dosometric nebuliser is described in International patent applications WO 004/045689 and WO 2004/045690 in the name of Profile Respiratory Systems Limited. These nebulisers are able to deliver the drug formulation to the user more efficiently by continually monitoring and predicting the user's breathing pattern and releasing the drug formulation only during the optimum phase of the user's breathing cycle, that is when the user is inhaling. For example, the nebuliser may monitor three successive breaths preceding the delivery dose and start delivering the drug formulation on the fourth breath and every breath thereafter. By targeting drug delivery to the optimal part of the user's breathing cycle, it is possible deliver the desired dose of a drug to the user whilst nebulising a significantly reduced volume of the drug solution.

It is desirable for the kits of the invention to include a nebuliser that calculates the amount of drug formulation released during each breathing cycle and has the capacity to stop drug delivery when the desired drug dose has been delivered to the user's airways. This ensures that the user always receives the correct dose and eliminates the risk of an overdose.

The nebulisers used in the invention preferably include control means which cause the nebuliser to initiate drug delivery and which regulate drug delivery. The control means are able to read data present on a data carrier (which may be integrated with or installed in the nebuliser), and respond to that data so that, in use, the correct dose of the drug formulation is delivered to the user. The control means may be linked to a display device that displays data present on the data carrier or other data.

As indicated, the data carrier may be supplied together with the UDVs for use in the nebuliser and may be, for example, a computer-readable disk of any type readable by the control means. Other forms of data carrier include a CMOS RAM. Preferably the data carrier bears one or more of data identifying the drug, formulation data (e.g. the amount or concentration of each component of the drug formulation present in each UDV), data identifying the batch number, data identifying the expiry date, dosage data (e.g. the dose to be present in each UDV), efficacy data (i.e. information relating to the physiological effect of the drug formulation, adverse effects or contraindications), and administration data (e.g. the recommended dose to be given to the user or the recommended frequency of administration).

It is also preferable that the data carrier bears a program for actuating drug delivery. This introduces a measure of security, in that in this embodiment, it would not be possible to use the nebuliser to administer a drug in the absence of a separate data carrier, which in turn would only be supplied with UDVs approved for use with the nebuliser.

The data (when provided as a separate integer from the nebuliser) carrier preferably bears data identifying the number of UDVs supplied with that data carrier. The control means can be adapted to read and amend this data when the nebuliser is used so that, after such use, the data carrier bears data identifying the number of remaining UDVs, i.e. unused UDVs supplied with the data carrier. More preferably the control means is designed so as not to permit actuation of drug delivery when the data identifying the number of remaining UDVs corresponds to all of the supplied UDVs having been used. This will prevent the nebuliser being used to dispense UDVs that are not associated with a valid data carrier. Thus, the control means and the data carrier interact to count down the number of remaining UDVs supplied with the data carrier and do not allow drug delivery after use of the last UDV. It also can ensure that other UDVs of greater volume, strength, or containing different drugs cannot be used in the nebuliser.

In another aspect, the invention provides a set of components for use in the kit of the invention comprising one or more UDVs and a data carrier, wherein the UDVs contain a pre-mixed drug formulation having a volume of less than 1ml and the data carrier bears data associated with the one or more UDVs.

A further aspect of the invention provides a data carrier for use in the kit of the invention, wherein the data carrier bears one or more of: data identifying the drug, formulation data, data identifying the batch number, data identifying the expiry date, dosage data, efficacy data, and administration data.

A further aspect of the invention provides a UDV for use in the kit of the invention, wherein the UDV contains a pre-mixed drug formulation having a volume of less than 1ml, and, preferably 0.5-1.0ml, wherein the drug formulation comprises Budesonide. A UDV for use according to the invention may comprise any suitable vessel suitable for storage of a pre-mixed drug formulation. UDVs may be of any known type and are of a size appropriate to contain the required volume of the drug formulation, preferably a pre-mixed and pre-measured single unit dose. UDVs according to the invention can be made of any suitable material, typically glass or plastics material.

The UDVs preferably contain a volume of a pre-mixed drug formulation, typically an aqueous solution or suspension. The drug formulation may comprise any drug suitable for administration as an aerosol. Suitable drugs include β2 agonists, steroids, anti-asthma drugs or drugs used in treatment of chronic obstructive pulmonary disease (COPD). Thus, the drug formulation may comprise one or more of Budesonide, Albuterol (also referred to as Salbutamol), Levalbuterol, Ipratropium, Oxitropium, Triotropium, Fenoterol, Terbutaline, Bambuterol, Eformoterol, Salmeterol, Fluticasone, Beclomethasone, Betamathasone, Cromoglycate (also referred to as Cromolyn), Nedocromil (and all their salts) either alone or in any combination of each. A preferred drug formulation comprises salbutamol (albuterol) and ipratropium bromide in combination. The drug formulations are preferably sterile.

The following Table illustrates specific concentrations of active ingredients that are typically prescribed, although other concentrations are known and prescribed.

| **ACTIVE** | **CONCENTRATION(S)** |
|---|---|
| Budesonide | 0.0125%, 0.025%, 0.05% |
| Albuterol | 0.083% |
| Ipratropium | 0.02% |
| Levalbuterol | 0.01%, 0.021%, 0.042% |
| Fluticasone | 0.025%, 0.1% |
| Cromoglycate | 1.0% |
| Albuterol/Ipratropium | 0.083%/0.17% |

UDVs of the invention generally contain a volume of a drug formulation of less than 1 ml. The volume may be, for example from 0.5ml to 1.0ml. Typically, UDVs according to the invention contain approximately one quarter of the volume of conventional UDVs, and therefore represent a saving in drugs and materials. As the UDVs are primarily for use with a high efficiency nebuliser, they do not need to contain the relatively high volumes of drug formulations used in conventional nebulisers. The UDVs of the invention need only contain a volume sufficient to fill the dead space in the internal volume of the nebuliser and to provide the desired dose. Minimal allowance need be made for loss of the drug formulation to atmosphere, swallowing or other wastage due to the efficiency of the nebulisers.

The invention further provides a method of using a nebuliser comprising:
providing a UDV containing a pre-mixed drug formulation;
in any order, transferring the drug formulation to the chamber of the nebuliser and installing a data carrier in the nebuliser; and
initiating drug delivery from the nebuliser,
wherein the nebuliser comprises control means for controlling the nebuliser to initiate drug delivery and the data carrier bears data associated with the UDV, whereby, in use, data is read by the control means and permits actuation of drug delivery.

According to this aspect of the invention, the UDVs may contain any suitable volume of the drug formulation. Preferably the volume is less than 1ml, as described for other aspects of the invention.

Typically UDVs for use in this aspect of the invention are supplied with a data carrier bearing data as described for other aspects of the invention. In use, the nebuliser chamber is opened, for example by removing the mouthpiece, though this may vary according to the type and model of nebuliser used. The UDV is then opened and the drug formulation within the UDV is emptied into the chamber. The chamber is then closed. Before use the data carrier is installed in the nebuliser, either before or after the chamber is filled with the drug formulation. The nebuliser is then activated to release the drug formulation. The user typically holds the nebuliser with its mouthpiece in their mouth or places a connected mask over the nose and mouth and start breathing. The control means of the nebuliser monitors the user's breathing pattern and starts releasing the drug formulation, as an aerosol, to coincide with, for example, the user's fourth inhalation. Drug delivery continues until the desired dose is delivered to the user, then ceases.

In another aspect, the invention provides a method of using a nebuliser comprising:
providing a UDV containing a pre-mixed drug formulation;
transferring the drug formulation to the chamber of the nebuliser;
initiating drug delivery from the nebuliser,
wherein the drug formulation has a volume of less than 1ml, preferably 0.5-1.0ml.

According to this aspect of the invention, the chamber of the nebuliser is opened, a UDV is unsealed and the drug formulation is transferred from the UDV to the chamber. The chamber is then closed and drug delivery is initiated. Again the nebuliser monitors the user's breathing pattern and drug release is timed to coincide with inhalation by the user and ceases when the desired dose has been delivered.

The methods of the invention may use any one or more kits, sets, nebulisers, control means, data carriers, UDVs, and drug formulations as described in respect of all other aspects of the invention.

The invention is now illustrated in specific embodiments by way of the following examples.

In these examples, UDVs are manufactured and filled under sterile conditions and provide formulations and strengths similar to (or identical to) solutions and suspensions for inhalation conventionally used (or any combination of two or more such formulations). These include the formulations published from time to time in both MIMS (Monthly Index of Medicinal Specialities) in the United Kingdom and in the "Orange Book" in the United States.

### Example 1

A formulation for use in kits according to the invention comprise Salbutamol (Albuterol sulfate) at a concentration of 0.083%, sodium chloride to adjust tonicity and sulphuric acid to adjust the pH to between 3.3 and 4.5.

The resulting solution is sterilised by filtration and then filled into recently formed sterile polyethylene UDVs which are then immediately sealed in one continuous operation. This process is commonly known as Blow Fill Seal and it is a recognised pharmaceutical industry standard for the manufacture of unit dose sterile liquid products. Each UDV contained 1ml of solution.

A data carrier consisting of a programmable microprocessor (in the form of recordable CMOS RAM or disc) is prepared and loaded with data related to the manufactured batch of Salbutamol solution. The loaded data includes the drug name, the concentration of each component, the final volume, the formulation batch number, the expiry date, the recommended dose and the number of UDVs supplied together with the data carrier. Details of the drug name, batch number and expiry date are also printed on one side of the data carrier to allow the data carrier to be identified other than by installing it in an appropriate nebuliser.

The data carrier and 20 UDVs are then packaged together for distribution and sale.

### Examples 2-8

Further UDVs are prepared as detailed in Example 1 except that the components of the drug formulation are as set out in the following Table and the data recorded on the data carrier relates to the formulation detailed in each example.

| **ACTIVE** | **CONCENTRATION(S)** |
|---|---|
| Budesonide | 0.0125%, 0.025%, 0.05% |
| Ipratropium | 0.02% |
| Levalbuterol | 0.01%, 0.021%, 0.042% |
| Fluticasone | 0.025%, 0.1% |
| Cromoglycate | 1.0% |
| Albuterol/Ipratropium | 0.083%/0.17% |

### Example 9

A high efficiency dosometric nebuliser, a pack of UDVs containing the drug formulation of Examples 1 - 9 and the accompanying data carrier are assembled and used to deliver the drug formulation to a user.

The data carrier is inserted into a slot for receiving the data carrier present on the front surface of the nebuliser, with its printed side facing outwards. The mouthpiece of the nebuliser is removed to reveal the filling chamber of the nebuliser and its cover. The clip retaining the cover is released and the cover removed in preparation for filling. One UDV is taken from the pack, opened by twisting off the top, and emptied into the filling chamber. The cover of the filling chamber is then replaced, the cover is secured with the clip, and the mouthpiece is replaced.

Following assembly, the nebuliser is switched on and the screen display indicates that the nebuliser is ready for use.

The user holds the nebuliser, with the mouthpiece in their mouth and begins breathing. The nebuliser monitors the user's first three breaths and predicts the user's fourth inhalation, during which delivery of the drug formulation commences. The nebuliser continues to predict each successive breath based on the preceding three breaths and to release drug during each successive inhalation until the user receives the recommended dose.

## Claims

1. A kit for administering a drug comprising
a nebuliser including control means for controlling the nebuliser to initiate drug delivery, and
one or more unit dosage vials (UDVs),
and wherein the UDVs contain a pre-mixed drug formulation, said formulation having a volume of less than 1ml, and wherein data storage means is provided which bears data associated with the one or more UDVs and is readable by the control means, whereby, in use, data is read by the control means and permits actuation of drug delivery.

2. A kit according to Claim 1, wherein the data storage means bears a program for actuating drug delivery.

3. A kit according to Claim 1 or 2, wherein the data storage means bears one or more of: data identifying the drug, formulation data, data identifying the batch number, data identifying the expiry date, dosage data, efficacy data, and administration data.

4. A kit according to any of Claims 1-3, wherein the data storage means bears data identifying the number of UDVs supplied with the data storage means and, in use, the control means amends this data when a UDV is used so that, after such use, the data storage means bears data identifying the number of remaining UDVs.

5. A kit according to Claim 4, wherein the control means is designed so as not to permit actuation of drug delivery when the data identifying the number of remaining UDVs is zero.

6. A kit according to any preceding claim wherein the data storage means is incorporated as part of the nebuliser.

7. A kit according to any of Claims 1 to 5, wherein the data storage means is provided as an element of the kit separate from the nebuliser.

8. A kit according to Claim 7 wherein the data storage means is adapted to be connected to the nebuliser to permit actuation of drug delivery.

9. A kit according to any of Claims 1-8, wherein the drug formulation comprises a β2 agonist, a steroid, an anti-asthma drug or a drug used in treatment of chronic obstructive pulmonary disease (COPD).

10. A kit according to Claim 9, wherein the drug formulation comprises Salbutamol (Albuterol), Budesonide, ipratropium bromide or sodium cromoglycate.

11. A kit according to Claim 9 or 10 wherein the drug formulation comprises two or more active ingredients in combination.

12. A kit according to Claim 11, wherein the drug formulation comprises Salbutamol (Albuterol) and ipratropium bromide in combination.

13. A kit according to any of Claims 1-9, comprising one of the following active ingredients in the concentrations stated
| **ACTIVE** | **CONCENTRATION(S)** |
|---|---|
| Budesonide | 0.0125%, 0.025%, 0.05% |
| Ipratropium | 0.02% |
| Levalbuterol | 0.01%, 0.021%, 0.042% |
| Fluticasone | 0.025%, 0.1% |
| Cromoglycate | 1.0% |
| Albuterol/Ipratropium | 0.083%/0.17% |

14. A kit according to any of Claims 1-13, wherein the drug formulation has a volume of 0.5-1.0 ml.

15. A kit according to any of Claims 1-14, wherein the nebuliser is an adaptive aerosol delivery nebuliser.
